# EUROPÄISCHE PATENTSCHRIFT

(11) **EP 1 117 387 B1**
(45) Veröffentlichungstag und Bekanntmachung des Hinweises auf die Patenterteilung: **12.01.2005**
(21) Anmeldenummer: 99948881.0
(22) Anmeldetag: 28.09.1999
(51) Int. Cl.: A61K 9/50

(54) **ÜBERZOGENE ARZNEIFORMEN MIT KONTROLLIERTER WIRKSTOFFABGABE**
COATED MEDICAMENT FORMS WITH CONTROLLED ACTIVE SUBSTANCE RELEASE
FORMES MEDICAMENTEUSES ENROBEES A LIBERATION CONTROLEE DU PRINCIPE ACTIF

(30) Priorität: 02.10.1998 DE 19845358
(43) Veröffentlichungstag der Anmeldung: 25.07.2001
(73) Patentinhaber: Röhm GmbH & Co. KG, 64293 Darmstadt (DE)
(72) Erfinder: PETEREIT, Hans-Ulrich, D-64291 Darmstadt (DE); BECKERT, Thomas, D-64297 Darmstadt (DE); LYNENSKJOLD, Eva, Bellevue, WA 98006 (US)
(86) Internationale Anmeldenummer: PCT/EP1999/007179
(87) Internationale Veröffentlichungsnummer: WO 2000/019984

(56) Entgegenhaltungen:
- EP-A- 0 068 191
- EP-A- 0 148 811
- EP-A- 0 225 085
- EP-A- 0 436 370
- EP-A- 0 640 341

## Beschreibung

Die Erfindung betrifft das Gebiet der überzogenen Arzneiformen mit kontrollierter Wirkstoffabgabe.

### Stand der Technik

EP-A 0 463 877 beschreibt pharmazeutische Zusammensetzungen mit verzögerter Wirkstofffreisetzung, bestehend aus einem Kern mit einem pharmazeutischen Wirkstoff und einem einschichtigen Überzugsfilm der ein wasserabweisendes Salz und ein wasserunlösliches Copolymer aus Ethylacrylat, Methylmethacrylat und Trimethylammoniumethlymethacrylat-Chlorid enthält. Das wasserabweisende Salz kann z. B. Ca- oder Mg-Stearat sein. Es werden sigmoide Freisetzungskurven erhalten.

EP-A 0 225 085, EP-A 0 122 077 und EP-A 0 123 470 beschreiben die Verwendung organischer Säure in Arzneimittelkernen, die mit verschiedenen Überzügen aus organischen Lösungen versehen werden. Es resultieren im wesentlichen sigmoide Freisetzungscharakteristiken. EP-A 0 436 370 beschreibt pharmazeutische Zusammensetzungen mit verzögerter Wirkstofffreisetzung, bestehend aus einem Kern mit einem pharmazeutischen Wirkstoff und einer organischen Säure und einem äußeren Überzugsfilm der durch wäßriges Sprühen aufgebracht wurde und ein Copolymer aus Ethylacrylat, Methylmethacrylat und Trimethylammoniumethlymethacrylat-Chlorid ist. Dabei werden ebenfalls sigmoide Freisetzungskurven erhalten. Die Verwendung der organischen Säuren in der Salzform wird in keinem der Zitate in Erwägung gezogen.

### Aufgabe und Lösung

Es wurde als Aufgabe gesehen, die aus EP-A 0 463 877 und EP-A 0 436 370 bekannten pharmazeutischen Präparationen weiterzuentwickeln. Dabei sollten unter anderem auch bereits bei relativ geringen Schichtdicken sigmoide Freisetzungskurven erhalten werden, die sonst nur mit höheren Aufträgen des Überzugsmittels erreicht werden können. Die Aufgabe wurde gelöst durch eine pharmazeutische Zubereitung bestehend aus
(a) einem Kern, enthaltend einen Wirkstoff, gegebenenfalls einen Träger und übliche pharmazeutische Zusatzstoffe, sowie das Salz einer organischen Säure, dessen Anteil am Kerngewicht 2,5 bis 97,5 Gew.-% ausmacht und
(b) einem äußeren Überzugsfilm, der aus einem oder mehreren (Meth)acrylat-Copolymeren sowie gegebenenfalls aus üblichen pharmazeutischen Hilfsstoffen besteht,
wobei 40 bis 100 Gew.-% der (Meth)acrylat-Copolymeren zu 93 bis 98 Gew.-% aus radikalisch polymerisierten C1- bis C4-Alkylestern der Acryl- oder der Methacrylsäure und 7 bis 2 Gew.-% (Meth)acrylat-Monomeren mit einer quaternären Ammoniumgruppe im Alkylrest bestehen und gegebenenfalls in einer Mischung vorliegen können mit
1 bis 60 Gew.-% einem oder mehreren weiteren, vom erstgenannten (Meth)acrylat-Copolymeren verschiedenen (Meth)acrylat-Copolymeren, die sich zu 85 bis 100 Gew.-% aus radikalisch polymerisierten C1- bis C4-Alkylestern der Acryl- oder der Methacrylsäure und gegebenenfalls bis zu 15 Gew.-% weiterer (Meth)acrylat-Monomere mit basischen Gruppen oder Säuregruppe im Alkylrest zusammensetzen.

### Ausführung der Erfindung

### Kerne (a)

Im einfachsten Fall kann der Kern nur aus dem Wirkstoff und dem Salz der organischen Säure zusammengesetzt sein, in der Regel enthält er zusätzlich einen Träger, z. B. ein Nonpareill, und pharmazeutische Hilfsstoffe, wie z. B. hochdisperse Kieselsäure oder Polyvinylpyrolidon (PVP).

Der Kern (a) besteht somit aus
- Wirkstoff bis 97,5 bis 2,5, bevorzugt 80 bis 5 Gew.%
- einem oder mehreren Salzen von organischen Säure 2,5 bis 97,5, bevorzugt 5 bis 80, insbesondere 10 bis 50 Gew. %
- pharmazeutische Hilfsstoffen, 0 - 95, bevorzugt 10 bis 50 Gew.%
- einen Träger 0 bis 95, bevorzugt 10 bis 50 Gew.-%

Die Kerne können z. B. durch direktes Verpressen, Extrusion und anschließende Ausrundung, feuchte oder trockene Granulation oder direkte Pelletierung (z.B. auf Tellern) oder durch Binden von Pulvern (Powder layering) auf wirkstoffreie Kugeln (Nonpareilles) oder wirkstoffhaltige Partikeln hergestellt werden.

Die zusätzlich zum Wirkstoff enthaltenen pharmazeutischen Hilfsstoffe können z. B. Bindemittel, wie Cellulose und deren Derivate, Polyvinylpyrolidon (PVP) , Gelatine, (Meth)acrylate, Stärke und deren Derivate oder Zucker sein.

Die Kerne können homogen sein oder einen schichtartigen Aufbau aufweisen, wobei sich der Wirkstoff bevorzugt in der äußern Schicht befindet.

### Salze organischer Säuren:

Die eingesetzten Salze organischer Säuren müssen toxikologisch unbedenklich und in Arzneimitteln verwendbar sein. Bevorzugt sind Alkalisalze (Ammonium, Lithium, Natrium, Kalium). Der bevorzugte Typ hängt von der speziellen Formulierung ab; neben der erfindungsgemäßen Funktionalität sind jedoch auch die pharmakologischen Effekte der Ionen zu berücksichtigen. Bevorzugt sind Salze schwacher organischer Säuren wie Citronensäure, Fumarsäure, Ameisensäure, Essigsäure, Maleinsäure, Weinsäure, Glutarsäure oder Milchsäure.

Besonders geeignet sind Natrium-Succinat, Natrium-Citrat und Natrium-Acetat. Die Verwendung der Essigsäure als Natrium-Acetat bietet den Vorteil, das nicht mit einer Flüssigkeit sondern mit einem Feststoff gearbeitet werden kann.

Der Typ der Säure kontrolliert die Steifheit Wirkstoffgabekurve insbesondere bei sigmoiden Freigabekurven.

In den erfindungsgemäßen Formulierungen liegen die Salze als äußere Schicht des Kern vor, gebunden durch Bindemittel. Sie werden aufgebracht durch Aufsprühen aus Lösung oder durch pulverförmigen Auftrag unter gleichzeitiger Zugabe von Bindemittellösung.

In Einzelfällen sind jedoch auch Varianten sinnvoll, in denen der Wirkstoff in Mischung mit den Salzen aufgetragen wird oder zwischen der Wirkstoffschicht und der Salzschicht eine Isolierschicht aufgetragen wird. Das Salz der organischen Säure kann auch zuletzt auf den Kern aufgetragen werden, so daß es die äußere Schicht bildet.

Der Anteil der Salze der organischen Säure(n) am Kerngewicht beträgt 2,5 Gew% bis 97,5 Gew.-%, bevorzugt 5 bis 80 Gew.-%, insbesondere 10 - 50 Gew.-%.

### Äußerer Überzugsfilm (b)

Der äußere Überzugsfilm, besteht aus einem oder mehreren (Meth)acrylat-Copolymeren sowie gegebenenfalls aus üblichen pharmazeutischen Hilfsstoffen, wie z. B. Weichmachern, Pigmenten, Porenbildnern, Netzmitteln, Trennmitteln etc..

Das Erfindungsprinzip beruht auf einer vermuteten Wechselwirkung zwischen dem Salz einer organischen Säure und einem (Meth)acrylat-Copolymeren, bestehend zu 93 bis 98 Gew.-% aus C1- bis C4-Alkylestern der Acryl- oder der Methacrylsäure und 7 bis 2 Gew.-% (Meth)acrylat Monomeren mit einer quaternären Ammoniumgruppe im Alkylrest. Um diese Wechselwirkung sicherzustellen muß das genannte (Meth)acrylat-Copolymere zu mindestens 40 Gew.-% am Aufbau des Überzugs beteiligt sein, um die gewünschte Wechselwirkung zu erreichen. Derartige (Meth)acrylat-Monomere sind handelsüblich und werden seit langem für retardierende Überzüge verwendet. Sie sind praktisch wasserunlöslich. Sie können allein oder Mischung mit anderen (Meth)acrylat-Copolymeren verwendet werden. Will man sigmoide Wirkstofffreisetzungcharakteristiken realisieren, so muß der Überzug (b) zu mindestens 80, bevorzugt zu 90 oder 100 Gew.-% aus dem genannten Copolymer-Typ bestehen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quarternären Ammoniumgruppen wird 2-Trimethylammoniumethlymethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-%-Trimethylammoniumethlymethacrylat-Chlorid aufgebaut sein.

Ein bevorzugtes Copolymer enthält 65 Gew.-% Methylmethacrylat, 30 Gew.-% Ethylacrylat und 5 Gew.-% 2-Trimethylammoniumethlymethacrylat-Chlorid aufgebaut sein (EUDRAGIT® RS)

### Mischungen von (Meth)acrylat-Copolymeren

Die Mischung des o. g. Copolymeren mit weiteren (Meth)acrylat-Copolymeren erlaubt die Einstellung individueller Wirkstoff-Freigabeprofile. So können statt sigmoider Kurven z. B. auch Profile mit sofortiger Freisetzung oder z. B. kontinuierliche Freisetzungsprofile oder Profile 0. (nullter) oder 1. Ordnung erhalten werden. Je nach Wirkstoffanforderung können auch Übergangsprofile der genannten Typen realisiert werden.

Erfindungsgemäß kann der äußere Überzugsfilm (b) deshalb auch eine Mischung des zu mindestens 40 Gew.-% vorhandenen (Meth)acrylat-Copolymeren, bestehend zu 93 bis 98 Gew.-% aus C1- bis C4-Alkylestern der Acryl- oder der Methacrylsäure und 7 bis 2 Gew.-% (Meth)acrylat Monomeren mit einer quaternären Ammoniumgruppe im Alkylrest, mit 1 - 60 Gew.-%, bevorzugt zu 40 - 60 Gew.-%, eines oder mehreren, bevorzugt einem, weiteren (Meth)acrylat-Copolymeren, das zu 85 bis 100 Gew.-% aus C1- bis C4-Alkylestern der Acryl- oder der Methacrylsäure und gegebenenfalls bis zu 15 Gew.-% weiterer (Meth)acrylat-Monomere mit funktionellen basischen Gruppen oder Säuregruppen im Alkylrest ausgebaut ist. Bei einem Gehalt von mehr als 15 Gew.-% basischen Gruppen oder Säuregruppen im Alkylrest werden die Wechselwirkungen der Komponenten untereinander in unerwünschter bzw. kaum vorhersehbarer Weise beeinflußt.

Ein geeignetes (Meth)acrylat-Copolymer für eine solche Mischung kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylat-Monomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (EUDRAGIT® RL).

Ein weiteres geeignetes (Meth)acrylat-Copolymer für eine Mischung besteht zu 95 bis 100 Gew.-% aus C1- bis C4-Alkylestern der Acryl- oder der Methacrylsäure und 0 - 5 Gew.-% aus Acryl- oder Methacrylsäure. Derartige (Meth)acrylat-Monomere sind handelsüblich.

### Herstellung der (Meth)acrylat-Copolymere allgemein

Die (Meth)acrylat-Copolymere werden in an sich bekannter Weise durch radikalische Substanz-, Lösungs-, Perl- oder Emulsionspolymerisation erhalten. Sie können als extrudiertes Granulat, gemahlenes Pulver, Lösung oder Dispersion vorliegen.

### Überzüge

Der Polymerauftrag hängt vor der Größe und Oberfläche der Kerne, von der Löslichkeit der Wirkstoffe und von dem angestrebten Freigabeprofil ab. Er liegt zwischen 5 und 80 Gew.% bezogen auf den Kern, bevorzugt zwischen 10 und 60%.

Die Überzüge können mehrschichtig oder als Mischung aufgetragen werden. Mischungen der Polymere erlauben die Einstellung bestimmter Steigungen in der zweiten Phase des Freigabeprofils. Der Gehalt an quaternären Ammoniumgruppen im Überzug regelt die Permeabilität und somit die Diffusionsgeschwindigkeit gelöster Substanzen (McGinity, Ed., Aqueous Polymeric Coatings for Pharmaceutical Dosage Forms, Marcel Dekker, Inc., Chapter 4, S .208-216). Je höher der Anteil an hydrophilen, quaternären Ammoniumgruppen, desto schneller ist die Freigabegeschwindigkeit. Man erhält so eine zusätzliche Steuermöglichkeit für die Wirkstoffabgabe in der zweiten Phase des Freigabeprofils.

### Weitere Zusatzstoffe

Sie dienen vor allem als Verarbeitungshilfsmittel und sollen ein sicheres und reproduzierbares Herstellungsverfahren sowie gute Langzeitlagerstabilität gewährleisten. Sie können die Permeabilität der Überzüge beeinflussen, was ggf. als zusätzlicher Steuerparameter genutzt werden kann.

### - Weichmacher:

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 4000 bis 20.000. Bevorzugte Weichmacher sind Triethylcitrat und Acetyltriethylcitrat.
Sind in der Regel Ester und bei Raumtemperatur flüssig:
Citrate, Phthalate, Sebacate, Rizinusöl

### - Antihaftmittel:

Diese Substanzen, die in der Regel lipophile Eigenschaften besitzen, werden den Sprühsuspensionen zugesetzt und verhindern eine Agglomeration der Kerne während der Befilmung. Bevorzugt werden Talkum, gemahlene Kieselsäure oder nicht ionische Emulgatoren mit einem HLB-Wert zwischen 3 und 8 eingesetzt. Die Mengen liegen zwischen 3 und 100 Gew % bezogen auf das Polymere
- Als weitere Zusätze können in an sich bekannter Weise z. B. Stabilisatoren, Farbstoffe, Antioxidantien, Netzmittel, Porenbildner, Pigmente, Glanzmittel etc. zugegeben werden.

### Auftrag des Filmüberzuges:

Auftragsverfahren erfolgt mittels Sprühauftrag aus organischer Lösung, oder wäßrigen Dispersionen durch Schmelzen oder durch direkten Pulverauftrag. Für die Ausführung ist dabei entscheidend, daß gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Wirkstoffe (Biologisch aktive Substanzen):

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichende thermische Stabilität besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
BroncholytikalAntiasthmatika, Cholinergika, Corticoide (Interna),
Dermatika, Diuretika, Enzyminhibitoren, Enzympräparate und
Transportproteine, Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

### Bevorzugte Wirkstoffe für verzögerte Wirkstoffabgabe sind:

Nifedipin, Diltiazem, Theophyllin, Diclofenac Na, Ketoprofen, Ibuprofen, Indometazin, Diltianzem, Ambroxol, Terbutalin, Vincamin, Propranolol, Pentoxyphyllin, Kodein, Morphin, Etilefrin, Carbamazepin bzw. deren therapeutische eingesetzte Salze.

### Applikationsformen und weitere Ausgestaltungen

Grundsätzlich könne die beschriebenen Arzneiformen direkt durch orale Applikation zur Anwendung kommen. Bevorzugt bei multipartikulären Formen (Multi Unit Dosage Forms) schließt man jedoch weitere Verarbeitungschritte an:

Die erfindungsgemäß hergestellte überzogenen Formen können als Einzeldosen in Gelatinekapseln und Beutel (Sachets) oder in geeignete Mehrdosenbehätter mit Dosiereinrichtung abgefüllt werden. Die Einnahme erfolgt in fester Form oder suspendiert in Flüssigkeiten.

Durch Verpressen erhält man aus Granulaten, ggf. nach Zumischung weiterer Hilfsstoffe, Tabletten die nach der Einnahme zerfallen und die Retardieten Untereinheiten freisetzen. Ebenso möglich ist die Einbettung von Agglomeraten in Polyethlenglykol oder Lipide zur Herstellung von Suppositorien oder vaginalen Arzneiformen.

Die Überzüge (b) können zusätzlich noch mit Überzügen des Standes der Technik kombiniert bzw. überzogen werden. Geeignet sind hierfür insbesondere (Meth)acrylat-Copolymere, die zu 10 bis 60 Gew.-% Methacrylsäure-Reste enthalten und im übrigen z. B. aus Methylmethacrylat und/oder Ethylacrylat aufgebaut sind (Typ EUDRAGIT® L oder S). Auf diese Weise können in Kombination mit den erfindungsgemäßen Formulierungen zusätzlich geschmacksisolierende Eigenschaften oder Formulierungen zur gezielte Freisetzungen im Colon realisiert werden.

### BEISPIELE

### Verwendete Copolymere

### Copolymer 1:

65 Gew.-% Methylmethacrylat, 30 Gew.-% Ethylacrylat und 5 Gew.-% 2-Trimethylammoniumethlymethacrylat-Chlorid (EUDRAGIT® RS).

### Copolymer 2:

60 Gew.-% Methylmethacrylat, 30 Gew.-% Ethylacrylat und 10 Gew.-% 2-Trimethylammoniumethlymethacrylat-Chlorid (EUDRAGIT® RL).

### Copolymer 3:

30 Gew.-% Ethylacrylat, 69 Gew.-% Methylmethacrylat und 1 Gew.-% Methacrylsäure (EUDRAGIT® NE 30D).

### Herstellung der überzogenen Theophyllin-Präparationen

Die Herstellung der wirkstoffhaltigen Pelletkerne erfolgt in an sich bekannter Weise in einem konventionellen Dragierkessel im Einstreuverfahren. Dabei rotieren die Trägerkerne (Nonpareilles) in dem Kessel und werden unter kontinuierlichem Befeuchten mit der Bindemittellösung (Sprühen, Düsendurchmesser 1 mm, Sprühdruck 0,5 bar, Sprühgeschwindigkeit 3 bis 6 g/min) mit der Pulvermischung (Einstreuen) zu Pellets aufgebaut. Anschließend wird das Produkt im Trockenschrank (24 Stunden bei 40 °C) getrocknet und die benötigte Teilchengrößenfraktion abgesiebt.

Es wurden in allen Beispielen Nonpareilles (0,5 - 0,6 mm) als Träger verwendet. Auf diese wurde Theophyllin als Wirkstoff, das jeweilig verwendete Salz der organischen Säure in unterschiedlichen Menge aufgebracht. Als pharmazeutische Hilfsstoffe wurden ein Polyvinylpyrolidon (Kollidon 25) in, und hochdisperse Kieseläure (Aerosil 200) oder Saccharose eingesetzt. Die Einstreuzeit betrug zwischen 77 und 142 min, bei 40 Kesselumdrehungen pro Minute.

Der Überzug wird nach bekanntem Verfahren in einem Wirbelschichtgerät aufgetragen. Es wurden wäßrige Dispersionen mit 30 Gew.-% Copolymergehalt eingesetzt. Zusätzlich wurden Triethylcitrat als Weichmacher und Talkum als Trennmittel verwendet.

### Messung der Theophyllin-Freisetzung

Die Bestimmung der Wirkstoffabgabe erfolgt in an sich bekannter Weise nach Pharm. Eur. in einem Paddle-Gerät bei einer Drehzahl von 100/min in gereinigtem Wasser oder Phosphatpuffer pH 6,8. Die Bestimmung der freigesetzten Wirkstoffmenge erfolgt photometrisch. Die Meßdauer richtet sich nach dem eingestellten Freigabeprofil und liegt zwischen 10 und 24 Stunden mit Meßpunkten nach jeweils 1 oder 2 Stunden. Nach Abschluß der Bestimmung wird die Testflüssigkeit mit dem verbleibenden Pelletresten homogenisiert und die enthaltene Wirkstoffmenge als 100%-Wert der Berechnung zu Grunde gelegt.

### Beispiel 1:

Freisetzungskurve zum Einfluß verschiedener Mengen (21, 32 und 43 Gew.-%) Natrium-Acetat bei Theophyllin/Natriumacetat-Kernen mit einem Überzugsauftrag von 30 Gew.-% Copolymer 1.

### Beispiel 2:

Freisetzungskurve zum Einfluß verschiedener Schichtdicken (20, 30 und 40 Gew.-%) von Copolymer 1 auf Theophyllin/Natriumacetat-Kernen.

### Beispiel 3:

Vergleich der Freigabe von Theophyllin aus Theophyllin/Natrium-Succinat (Na-Succinat) und Theophyllin/Bernsteinsäure-Präparationen mit 42 und 60 Gew.-% Überzugsauftrag von Copolymer 1.

### Beispiel 4:

Freisetzungskurve zum Einfluß verschiedener Schichtdicken ( 10, 20, 30 und 40 Gew.-%) einer Mischung (1:1) von Copolymer 1 und Copolymer 2 auf Theophyllin/Natriumacetat-Kernen.

### Beispiel 5:

Freisetzungskurve zum Einfluß verschiedener Schichtdicken (10, 20, 30 und 40 Gew.-%) einer Mischung (1:1) von Copolymer 1 und Copolymer 3 auf Theophyllin/Natriumacetat-Kernen.

Die Rezepturen für die Beispiele 1 bis 5 sind in der folgenden Tabelle zusammengefaßt (Alle Angaben in g):

| **Beispiel** | **1** | **2** | **3** | **4** | **5** |
|---|---|---|---|---|---|
| **KERNE 1) Einstreugemisch** | | | | | |
| Nonpareilles | 700 | 700 | 700 | 700 | 700 |
| Theophyllin | 901/675/450 | 675 | 420 | 675 | 675 |
| Na-Acetat | 450/675/901 | 675 | - | 675 | 675 |
| Na-Succinat/ Bernstein-Sre. | | | 960 / 700 | | |
| Kollidon 25 | 42 | 42 | 43 / 35 | 42 | 42 |
| Aerolsil 200 | 7 | 7 | 716 | 7 | 7 |

| **2) Bindemittel- Lösung** | | | | | |
|---|---|---|---|---|---|
| Kollidon 30 | 20 | 20 | - | 20 | 20 |
| Saccharose | - | - | 276/97 | - | - |
| Ethanol | - | - | 135/28 | - | - |
| Wasser | 380 | 380 | 430 / 85 | 380 | 380 |

| **KERNE + ÜBERZÜGE** | | | | | |
|---|---|---|---|---|---|
| Menge der überzogene Kerne | 500 | 500 | 500 | 500 | 500 |
| Copolymer 1-Dispersion 30 %-ig | 500 | 333/500/667 | 1005 | 83/167/250/ 333 | 83/167/250/ 333 |
| Copolymer 2-Dispersion 30 %-ig | - | - | - | 125/167/250/ 333 | - |
| Copolymer 3-Dispersion 30 %-ig | - | - | - | - | 83/167/250/ 333 |
| Triethylcitrat | 30 | 27/40/53 | 38 | 10/20/30/40 | 10/20/30/40 |
| Talkum | 75 | 50/75100 | 159 | 25/50/75/100 | 25/50/75/100 |
| Wasser | 670 | 447/670/893 | 1179 | 225/447/670/ 893 | 225/447/670/ 893 |

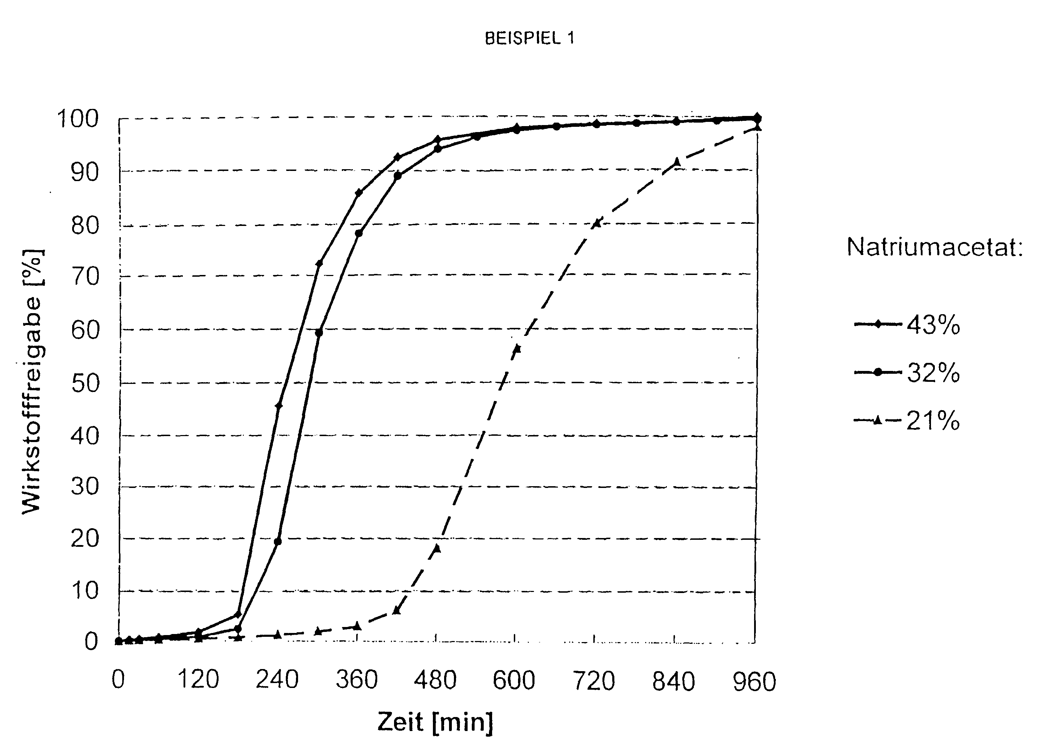

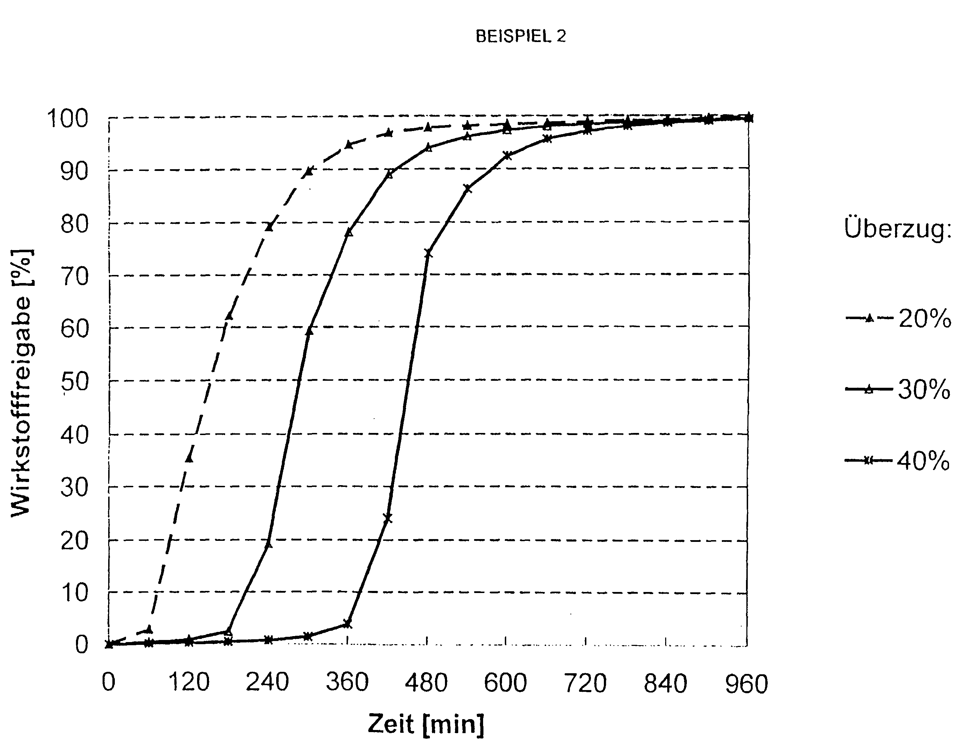

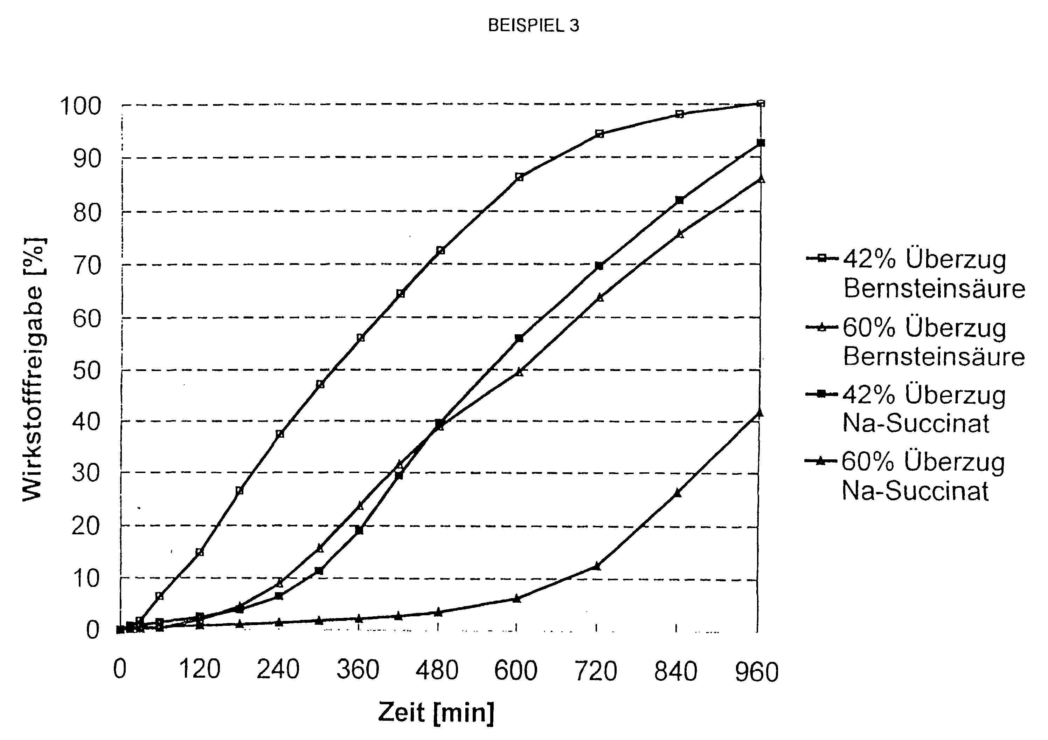

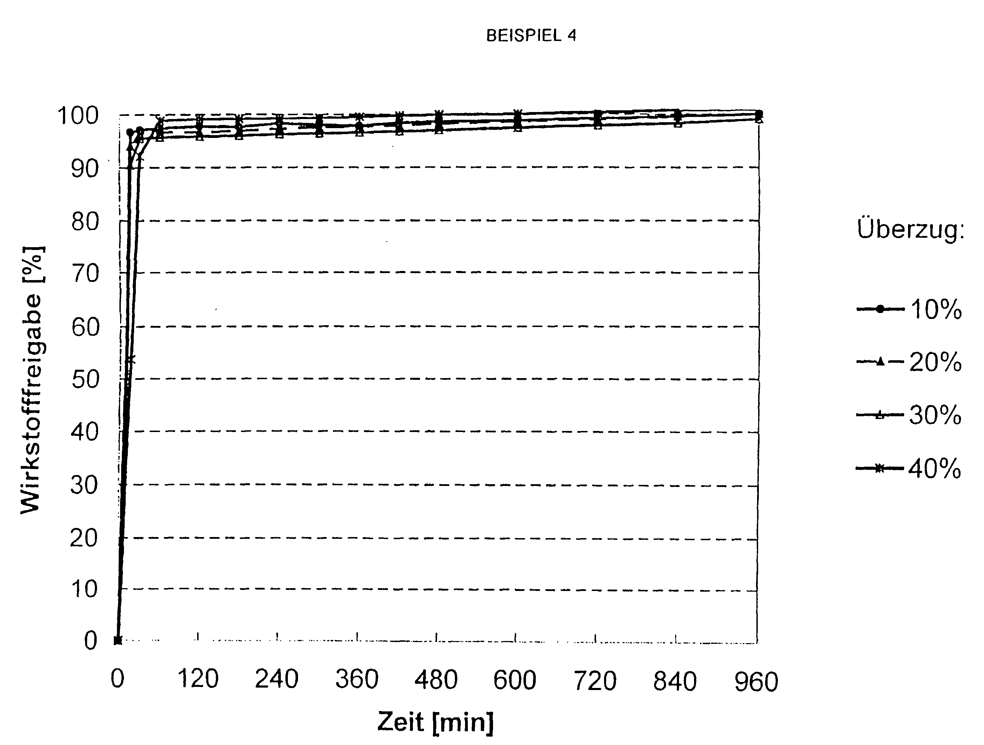

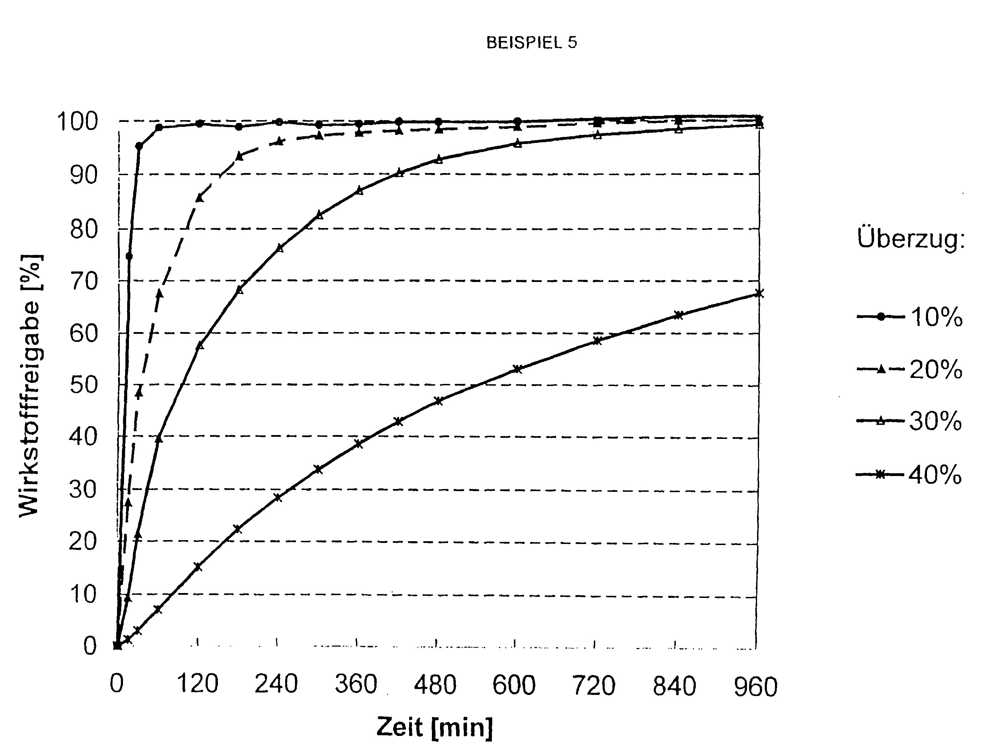

## Patentansprüche

1. Pharmazeutische Zubereitung bestehend aus
(a) einem Kern, enthaltend einen Wirkstoff, gegebenenfalls einen Träger und übliche pharmazeutische Zusatzstoffe, sowie das Salz einer organischen Säure, dessen Anteil am Kerngewicht 2,5 bis 97,5 Gew.-% ausmacht und
(b) einem äußeren Überzugsfilm, der aus einem oder mehreren (Meth)acrylat-Copolymeren sowie gegebenenfalls aus üblichen pharmazeutischen Hilfsstoffen besteht,
wobei 40 bis 100 Gew.-% der (Meth)acrylat-Copolymeren zu 93 bis 98 Gew.-% aus radikalisch polymerisierten C1- bis C4-Alkylestern der Acryl- oder der Methacrylsäure und 7 bis 2 Gew.-% (Meth)acrylat-Monomeren mit einer quaternären Ammoniumgruppe im Alkylrest bestehen und gegebenenfalls in einer Mischung vorliegen können mit
1 bis 60 Gew.-% einem oder mehreren weiteren, vom erstgenannten (Meth)acrylat-Copolymeren verschiedenen (Meth)acrylat-Copolymeren, die sich zu 85 bis 100 Gew.-% aus radikalisch polymerisierten C1- bis C4-Alkylestern der Acryl- oder der Methacrylsäure und gegebenenfalls bis zu 15 Gew.-% weiterer (Meth)acrylat-Monomere mit basischen Gruppen oder Säuregruppe im Alkylrest zusammensetzen.

2. Zubereitung nach Anspruch 1, **dadurch gekennzeichnet, daß** der (Meth)acrylat-Copolymer-Anteil des äußeren Überzugsfilms (b) eine Mischung ist aus
99 bis 40 Gew.-% eines (Meth)acrylat-Copolymeren, das zu 93 bis 98 Gew.-% aus C1- bis C4-Alkylestern der Acryl- oder der Methacrylsäure und 7 bis 2 Gew.-% (Meth)acrylat-Monomeren mit einer quaternären Ammoniumgruppe im Alkylrest besteht, und
1 - 60 Gew.-% eines (Meth)acrylat-Copolymeren, das zu 85 bis weniger als 92 Gew.-% aus C1- bis C4-Alkylestern der Acryl- oder der Methacrylsäure und mehr als 7 bis 15 Gew.-% (Meth)acrylat-Monomeren mit einer quaternären Ammoniumgruppe im Alkylrest aufgebaut ist.

3. Zubereitung nach Anspruch 1, **dadurch gekennzeichnet, daß** der (Meth)acrylat-Copolymer-Anteil des äußeren Überzugsfilms (b) eine Mischung ist aus
99 bis 40 Gew.-% eines (Meth)acrylat-Copolymeren, das zu 93 bis 98 Gew.-% aus C1- bis C4-Alkylestern der Acryl- oder der Methacrylsäure und 7 bis 2 Gew.-% (Meth)acrylat Monomeren mit einer quaternären Ammoniumgruppe im Alkylrest besteht, und
1 - 60 Gew.-% eines (Meth)acrylat-Copolymeren, das zu 95 bis 100 Gew.-% aus C1- bis C4-Alkylestern der Acryl- oder der Methacrylsäure und 0 - 5 Gew.-% aus Acryl- oder Methacrylsäure aufgebaut ist.

4. Zubereitung nach einem oder mehreren der Ansprüche 1 bis 3, **dadurch gekennzeichnet, daß** als (Meth)acrylat-Monomer mit einer quaternären Ammoniumgruppe im Alkylrest Trimethylammoniumethlymethacrylat-Chlorid verwendet wird.

5. Zubereitung nach einem oder mehreren der Ansprüche 1 bis 4, **dadurch gekennzeichnet, daß** als Salz der organischen Säure Ammonium-, Lithium-, Natrium- oder Kalium-Salze der Citronensäure, Fumarsäure, Ameisensäure, Essigsäure, Maleinsäure, Bernsteinsäure, Weinsäure, Glutarsäure oder Milchsäure verwendet werden.

6. Zubereitung nach einem oder mehreren der Ansprüche 1 bis 5, **dadurch gekennzeichnet, daß** sie zu Tabletten verpreßt ist.

7. Zubereitung nach einem oder mehreren der Ansprüche 1 bis 5, **dadurch gekennzeichnet, daß** sie von einer Gelatinekapsel umhüllt ist.

8. Zubereitung nach einem oder mehreren der Ansprüche 1 bis 6, **dadurch gekennzeichnet, daß** sie zusätzlich mit einem (Meth)acrylat-Copolymer, welches 10 - 60 Gew.-% Methacrylsäurereste enthält, umhüllt ist.

9. Zubereitung nach einem oder mehreren der vorhergehenden Ansprüche, **dadurch gekennzeichnet, daß** das Salz der organischen Säure die äußere Schicht des Kerns bildet.

## Claims

1. Pharmaceutical preparation consisting of
(a) a core containing an active substance, optionally a carrier and conventional pharmaceutical additives as well as the salt of an organic acid the proportion of which makes up 2.5 to 97.5 wt.% of the weight of the core and
(b) an outer coating film which consists of one or more (meth)acrylate copolymers and optionally conventional pharmaceutical excipients,
wherein 40 to 100 wt.% of the (meth)acrylate copolymers consist of 93 to 98 wt.% of radically polymerised C₁ to C₄-alkyl esters of acrylic or methacrylic acid and 7 to 2 wt.% of (meth)acrylate monomers with a quaternary ammonium group in the alkyl group and may optionally be present in admixture with
1 to 60 wt.% of one or more other (meth)acrylate copolymers different from the first-mentioned methacrylate copolymer, consisting of 85 to 100 wt.% of radically polymerised C₁- to C₄-alkyl esters of acrylic or methacrylic acid and optionally up to 15 wt.% of other (meth)acrylate monomers with basic groups or an acid group in the alkyl group.

2. Preparation according to claim 1, **characterised in that** the (meth)acrylate copolymer fraction of the outer coating film (b) is a mixture of
99 to 40 wt.% of a (meth)acrylate copolymer which consists of 93 to 98 wt.% of C₁- to C₄-alkyl esters of acrylic or methacrylic acid and 7 to 2 wt.% of (meth)acrylate monomer with a quaternary ammonium group in the alkyl group, and
1 to 60 wt.% of a (meth)acrylate copolymer which is made up of 85 to less than 92 wt.% of C₁- to C₄-alkyl esters of acrylic or methacrylic acid and more than 7 to 15 wt.% of (meth)acrylate monomers with a quaternary ammonium group in the alkyl group.

3. Preparation according to claim 1, **characterised in that** the (meth)acrylate copolymer fraction of the outer coating film (b) is a mixture of
99 to 40 wt.% of a (meth)acrylate copolymer which consists of 93 to 98 wt.% of C₁- to C₄-alkyl esters of acrylic or methacrylic acid and 7 to 2 wt.% of (meth)acrylate monomer with a quaternary ammonium group in the alkyl group, and
1 to 60 wt.% of a (meth)acrylate copolymer which is made up of 95 to 100 wt.% of C₁- to C₄-alkyl esters of acrylic or methacrylic acid and 0 to 5 wt.% of acrylic or methacrylic acid.

4. Preparation according to one or more of claims 1 to 3, **characterised in that** trimethyl ammonium ethyl methacrylate chloride is used as the (meth)acrylate monomer having a quaternary ammonium group in the alkyl group.

5. Preparation according to one or more of claims 1 to 4, **characterised in that** ammonium, lithium, sodium or potassium salts of citric acid, fumaric acid, formic acid, acetic acid, maleic acid, succinic acid, tartaric, glutaric or lactic acid are used as the salt of an organic acid.

6. Preparation according to one or more of claims 1 to 5, **characterised in that** it is compressed to form tablets.

7. Preparation according to one or more of claims 1 to 5, **characterised in that** it is surrounded by a gelatine capsule.

8. Preparation according to one or more of claims 1 to 6, **characterised in that** it is additionally surrounded by a (meth)acrylate copolymer which contains 10 to 60 wt.% of methacrylic acid groups.

9. Preparation according to one or more of the preceding claims, **characterised in that** the salt of the organic acid forms the outer layer of the core.

## Revendications

1. Préparation pharmaceutique constituée
(a) d'un noyau contenant une substance active, éventuellement un porteur et des additifs pharmaceutiques, ainsi que le sel d'un acide organique, dont la proportion par rapport au poids du noyau représente de 2,5 à 97,5 % en poids, et
(b) d'une pellicule d'enrobage externe qui est constituée d'un ou plusieurs copolymères de (méth)acrylate ainsi qu'éventuellement d'adjuvants pharmaceutiques usuels,
40 à 100 % en poids des copolymères de (méth)acrylate consistant à raison de 93 à 98 % en poids en esters alkyliques en C₁-C₄ de l'acide acrylique ou de l'acide méthacrylique, polymérisés par voie radicalaire, et à raison de 7 à 2 % en poids en monomères (méth)acrylate comportant un groupe ammonium quaternaire dans le reste alkyle et pouvant éventuellement se trouver en un mélange avec
1 à 60 % en poids d'un ou plusieurs autres copolymères de (méth)acrylate, différents du copolymère de (méth)acrylate cité en premier, qui se composent à raison de 85 à 100 % en poids d'esters alkyliques en C₁-C₄ de l'acide acrylique ou de l'acide méthacrylique, polymérisés par voie radicalaire, et éventuellement à raison de jusqu'à 15 % en poids d'autres monomères de (méth)acrylate comportant des groupes basiques ou un groupe acide dans le reste alkyle.

2. Préparation selon la revendication 1,
**caractérisée en ce que**
la fraction copolymère de (méth)acrylate de la pellicule d'enrobage externe (b) est un mélange constitué
de 99 à 40 % en poids d'un copolymère de (méth)acrylate qui consiste à raison de 93 à 98 % en poids en esters alkyliques en C₁-C₄ de l'acide acrylique ou de l'acide méthacrylique et à raison de 7 à 2 % en poids en monomères (méth)acrylate comportant un groupe ammonium quaternaire dans le reste alkyle, et
de 1 à 60 % en poids d'un copolymère de (méth)acrylate qui consiste à raison de 85 à moins de 92 % en poids en esters alkyliques en C₁-C₄ de l'acide acrylique ou de l'acide méthacrylique et à raison de plus de 7 à 15 % en poids en monomères (méth)acrylate comportant un groupe ammonium quaternaire dans le reste alkyle.

3. Préparation selon la revendication 1,
**caractérisée en ce que**
la fraction copolymère de (méth)acrylate de la pellicule d'enrobage externe (b) est un mélange constitué
de 99 à 40 % en poids d'un copolymère de (méth)acrylate qui consiste à raison de 93 à 98 % en poids en esters alkyliques en C₁-C₄ de l'acide acrylique ou de l'acide méthacrylique et à raison de 7 à 2 % en poids en monomères (méth)acrylate comportant un groupe ammonium quaternaire dans le reste alkyle, et
de 1 à 60 % en poids d'un copolymère de (méth)acrylate qui consiste à raison de 95 à 100 % en poids en esters alkyliques en C₁-C₄ de l'acide acrylique ou de l'acide méthacrylique et à raison de 0 à 5 % en poids en acide acrylique ou méthacrylique.

4. Préparation selon une ou plusieurs des revendications 1 à 3,
**caractérisée en ce que**
du chlorure de méthacrylate de triméthylammoniuméthyle est utilisé en tant que monomère (méth)acrylate comportant un groupe ammonium quaternaire dans le reste alkyle.

5. Préparation selon une ou plusieurs des revendications 1 à 4,
**caractérisée en ce que**
comme sel de l'acide organique on utilise les sels d'ammonium, de lithium, de sodium ou de potassium de l'acide citrique, fumarique, formique, acétique, maléique, succinique, tartrique, glutarique ou lactique.

6. Préparation selon une ou plusieurs des revendications 1 à 5,
**caractérisée en ce qu'**
elle est pressée en comprimés.

7. Préparation selon une ou plusieurs des revendications 1 à 5,
**caractérisée en ce qu'**
elle est entourée d'une capsule de gélatine.

8. Préparation selon une ou plusieurs des revendications 1 à 6,
**caractérisée en ce qu'**
elle est en outre enrobée d'un copolymère de (méth)acrylate qui contient de 10 à 60 % en poids de radicaux méthacryloyle.

9. Préparation selon une ou plusieurs des revendications précédentes,
**caractérisée en ce que**
le sel de l'acide organique constitue la couche externe du noyau.
